**Europäisches Patentamt**

**European Patent Office**

**Office européen des brevets**

(19)

(11) Numéro de publication: **0 210 081**
**B1**

(12) **FASCICULE DE BREVET EUROPEEN**

(45) Date de publication du fascicule du brevet:
**01.03.89**

(21) Numéro de dépôt: **86401089.7**

(22) Date de dépôt: **23.05.86**

(51) Int. Cl.⁴: **G 01 N 25/22**, G 01 N 33/22

(54) Procédé et installation pour la mesure du pouvoir calorifique de gaz combustibles.

(30) Priorité: **18.06.85 FR 8509238**

(43) Date de publication de la demande:
**28.01.87 Bulletin 87/5**

(45) Mention de la délivrance du brevet:
**01.03.89 Bulletin 89/9**

(84) Etats contractants désignés:
**BE DE GB IT NL**

(56) Documents cités:
**EP-A- 0 076 759**
**DE-C- 683 724**
**US-A- 2 026 179**
**US-A- 2 026 180**

(73) Titulaire: **SOCIETE NATIONALE ELF AQUITAINE (PRODUCTION), Tour Elf 2, Place de la Coupole La Défense 6, F-92400 Courbevoie (FR)**

(72) Inventeur: **Mondeil, Lucien, rue des Chènes, F-64160 Serres Morlaas (FR)**
Inventeur: **Robert, François, villa Copacabana avenue de Trespoey Prolongée, F-64320 Bizanos (FR)**

(74) Mandataire: **Epstein, Henri, 7 Rue du Temple, F-95880 Enghien les Bains (FR)**

ACTORUM AG

## Description

La présente invention se rapporte à un procédé de mesure en continu du pouvoir calorifique supérieur d'un gaz combustible au moyen d'un calorimètre comportant autour d'une cheminée centrale logeant un brûleur et des moyens de son alimentation à pression et débit déterminés deux cellules annulaires et concentriques, la paroi externe de la cellule externe étant pourvue en moyens d'échange de chaleur pour la régulation de sa température, lesquelles cellules concentriques séparées par une cloison annulaire parallèle aux parois de cellules sont reliées à un capteur de pressions différentielles.

Un calorimètre de ce type est connu du brevet français n° 81 18722 au nom conjoint de ONERA et de la déposante. Dans un tel calorimètre, les cellules interne et externe ont des épaisseurs faibles par rapport à leurs longueurs, ce qui permet de considérer que le gradient de température s'installant dans ces cellules est sensiblement normal aux parois, c'est-à-dire sensiblement radial, les cellules étant concentriques et annulaires. De ce fait, les courants de convection sont pratiquement inexistants et le flux thermique traverse le calorimètre en direction sensiblement radiale sans être perturbé par des mouvements ascendants et descendants du type de convection.

La mesure du flux thermique s'effectue au moyen d'un thermomètre différentiel à gaz basé sur le phénomène que, lorsque deux cellules remplies d'un même gaz et séparées par une cloison présentant une certaine résistance thermique sont traversées radialement par un flux thermique induisant une élévation de température dans les cellules, la différence de l'intégrale volumique des températures dans chaque cellule est proportionnelle au flux calorifique dégagé par la combustion au niveau du brûleur d'un gaz comburant et donc la différence des pressions existant dans chaque cellule est elle même proportionnelle à ce flux selon l'égalité:

$$\triangle P = K_1 \triangle \theta = K_2 \varphi, \text{ où}$$

$\triangle \theta$ est la différence de températures moyennes dans le cellules et $\varphi$ le flux calorifique dû au gaz carburant.

Toutefois, lorsqu'il s'agit de mesurer le pouvoir calorifique supérieur d'un gaz, à savoir le flux total émis par la combustion de ce gaz, il faut veiller à ce que le bilan thermique de l'opération prenne très exactement en compte en totalité la chaleur latente de condensation de l'eau formée par la combustion du gaz, et rien que cette chaleur latente, et ne soit pas faussé par des phénomènes parasites, tels que la prise en compte de la chaleur latente de condensation de l'eau qu'aurait contenu et abandonné au cours de l'opération l'air comburant initial ou de la non condensation d'une partie de l'eau formée par la combustion et due à la non saturation de l'air de combustion.

Dans ce cas, on peut poser:

$$\triangle P = K_3 \cdot q_g \cdot PCS_g, \text{ où}$$

$q_g$ est le débit du gaz combustible et
$PCS_g$ le pouvoir calorifique supérieur du gaz,
à condition qu'il n'y ait pas d'erreur d'échange.

Le procédé selon l'invention est donc caractérisé en ce qu'on maintient le rapport de la température de sortie de gaz brûlés à celle de gaz d'entrée sensiblement voisin de 1 et le degré de saturation de l'air comburant d'alimentation du brûleur à la limite de la saturation en eau, de façon à prendre en compte en totalité la chaleur latente de condensation de l'eau formée par la combustion et de mesurer le flux calorifique total émis par la combustion du gaz.

En effet, le maintien de la température de sortie de gaz brûlés très proche de la température d'entrée de gaz d'alimentation et le maintien du degré de saturation de l'air à la limite de la saturation s'opposent efficacement à toute erreur de bilan thermique qui pourrait être due

— soit à un apport d'humidité intempestif dans l'air comburant amenant un excès de condensation,

— soit à un défaut de saturation de l'air comburant amenant un manque de condensation,

— soit à un mauvais rapport des températures entrée/sortie de gaz amenant une surcondensation ou une souscondensation.

En maintenant le rapport de températures de gaz voisin de 1, on annule l'influence de l'eau contenu dans l'air, de sorte que c'est la même saturation qu'on retrouve dans le gaz de sortie. Ainsi, on ne fait condenser que l'eau formée par la combustion dont la chaleur latente est prise en compte dans le calorimètre.

De préférence, le rapport de la température de sortie de gaz brûlés à celle de gaz d'entrée est maintenu à une valeur légèrement supérieure à 1, par exemple pouvant varier entre 1,02 et 1,05, pour que, malgré la contraction du volume de gaz à la suite de la combustion, la totalité de la vapeur d'eau apportée par l'air comburant saturé soit conservée à l'état de vapeur dans les gaz brûlés de sortie, sans subir de condensation et ne se trouve condensée que la quantité exacte d'eau formée par la combustion.

En effet, si le volume de gaz réduit à la suite de la combustion gardait la même température, par exemple 40°C, que sa température d'entrée, on aurait une condensation d'eau supplémentaire pour que la saturation en eau soit conservée, ce qui fausserait le résultat de la mesure. En maintenant la température de sortie de gaz à une valeur légèrement supérieure à celle d'entrée de gaz, par exemple à 41,5°C, on évite ce supplément parasitaire d'eau condensée, qui passant pour de l'eau formée au cours de la combustion fausserait les résultats.

Ce rapport sera déterminé par le calcul à partir de données de débits de gaz et d'air, de la température d'entrée et des tables de pressions partielles de vapeur d'eau, de façon à ce que la température de sortie de gaz brûlés soit telle, que soit évité tout supplément de condensation due à la contraction du volume de gaz.

Une autre erreur qu'il s'agit d'éviter de commettre est l'erreur d'échange thermique variable dans le temps. En effet, lorsque le pouvoir calorifique supérieur du gaz varie, par exemple d'un gaz de référence à un gaz échantillon, les pertes thermiques varient également dans le temps et faussent la mesure. Pour annuler de tels effets, on opère selon l'invention à flux thermique constant par recours à une méthode de zéro qui permet de plus de s'affranchir des non linéarités éventuelles du capteur de pression servant à mesurer la valeur du flux.

En conséquence, selon une autre caractéristique de l'invention, le procédé est caractérisé en ce qu'on alterne les périodes de mesure du pouvoir calorifique du gaz échantillon $PCS_g$ avec les périodes d'étalonnage par la mesure du pouvoir calorifique connu d'un gaz de référence $PCS_r$ en effectuant les mesures des flux calorifiques selon une méthode de zéro, en ramenant pour chaque mesure la valeur du flux calorifique à une valeur déterminée constante par apport d'une énergie calorifique complémentaire $W_g$ et $W_r$ par une résistance additionnelle de chauffe, de façon à ce que, les débits de gaz $q_g$ et $q_r$ étant constants, en égalisant les flux calorifiques émis en phase d'étalonnage et en phase de mesure selon l'équation:

$$q_r \cdot PCS_r + W_r = q_g \cdot PCS_g + W_g,$$

la valeur du pouvoir calorifique supérieur du gaz échantillon

$$PCS_g = PCS_r + \frac{W_r - W_g}{q_g}$$

s'exprime en fonction de valeurs mesurées d'énergies calorifiques complémentaires d'apport extérieur $W_r$ et $W_g$, s'affranchissant ainsi des erreurs dues aux non linéarités du capteur de pressions différentielles.

Ainsi, l'égalité des flux calorifiques est réalisée en pilotant la puissance W et la résistance additionnelle pour maintenir constant le signal du capteur de pression différentielle.

Selon un mode de réalisation, on amène dans la phase d'étalonnage la valeur du flux total transmis à une valeur constante $\varphi_c$ en ajoutant au flux émis par la combustion du gaz de référence $\varphi_{cr}$ un complément d'énergie calorifique $W_r$ et en maintenant le rapport des températures de sortie de gaz brûlés et celle des gaz d'entrée par les moyens d'échange de chaleur dont est pourvue la paroi externe de la cellule externe à une valeur voisine de 1 et, dans la phase de mesure, on ramène la valeur du flux total transmis à la même valeur $\varphi_c$ par apport complémentaire d'énergie calorifique $W_g$ s'ajoutant au flux émis par la combustion du gaz à mesurer $\varphi_{cg}$.

De préférence, l'égalité des flux réalisée au cours de l'étalonnage et au cours de la mesure est contrôlée par les mesures de pressions différentielles $\triangle P$ du capteur relié aux deux cellules du calorimètre, de façon à maintenir $\triangle P$ à une valeur constante de consigne au cours des deux mesures.

La constance du débit volumique de gaz combustible pendant les phases d'étalonnage et de mesure est assurée par l'emploi d'une pompe volumétrique de précision, alimentée en gaz à pression régulée, immergée dans un bain de température régulée et dont la vitesse est pilotée par une alimentation électronique de très haute stabilité, la connaissance précise de la pression, de la température, de la vitesse et de la cylindrée de la pompe assurant la connaissance précise du débit de gaz.

La présente invention a également pour objet une installation de mesure en continu du pouvoir calorifique supérieur d'un gaz combustible comprenant un calorimètre comportant autour d'une cheminée centrale logeant un brûleur et des moyens de son alimentation en gaz combustible et en air comburant à pression et débit déterminés, deux cellules annulaires et concentriques, la paroi externe de la cellule externe étant pourvue de moyens d'échange de chaleur, lesdites cellules concentriques séparées par une cloison annulaire parallèle aux parois de cellules sont reliées à un capteur de pression différentielles, pour la mise en oeuvre du procédé décrit ci-dessus et qui est caractérisée en ce que la cheminée centrale est fermée au sommet où débouche l'entrée d'un serpentin qui entoure la paroi externe de la cheminée et débouche à son extrémité opposée à l'extérieur du calorimètre pour l'évacuation des gaz brûlés, une sonde de température étant logée au contact des gaz brûlés de sortie et une autre sonde au contact des gaz d'entrée, lesdites sondes étant reliées à une unité de calcul pour le traitement de leurs signaux, ladite unité de calcul étant munie de moyens pour actionner un circuit de chauffe de l'échangeur dont est pourvue la paroi externe de la cellule externe.

De préférence, la partie inférieure du serpentin est logée dans un échangeur de chaleur muni de moyens de circulation à contre-courant pour les gaz d'alimentation du brûleur.

Par une telle disposition, on contribue à favoriser la constance d'un rapport déterminé voisin de 1 des températures de gaz de sortie et d'entrée.

Selon une autre caractéristique de l'installation, une pompe volumétrique reliée, d'une part à une conduite d'arrivée de gaz de référence et du gaz échantillon munie d'un régulateur à pression constante et, d'autre part, un brûleur du calorimètre et un humidificateur d'air relié, d'une part, à une conduite d'arrivée d'air munie d'un régulateur de débit et, d'autre part, au tube central du calorimètre, sont plongés dans un bain thermostaté.

Selon une autre caractéristique de l'installation, une résistance électrique de chauffe est logée dans la cheminée centrale du calorimètre et les deux cellules du calorimètre sont reliées par des tubulures à un capteur de pression différentielles associé à une unité de calcul munie de moyens pour actionner un circuit de commande de la puissance fournie à ladite résistance de chauffe.

Afin d'éviter les pertes de flux thermique par conductance thermique, on fait de préférence déboucher le tube alimentant le brûleur dans la partie supérieure de la cheminée centrale.

D'autres particularités de l'invention apparaîtront à la lumière de la description d'un mode de réalisation de l'installation présenté à titre d'exemple et illustré par des dessins, dont

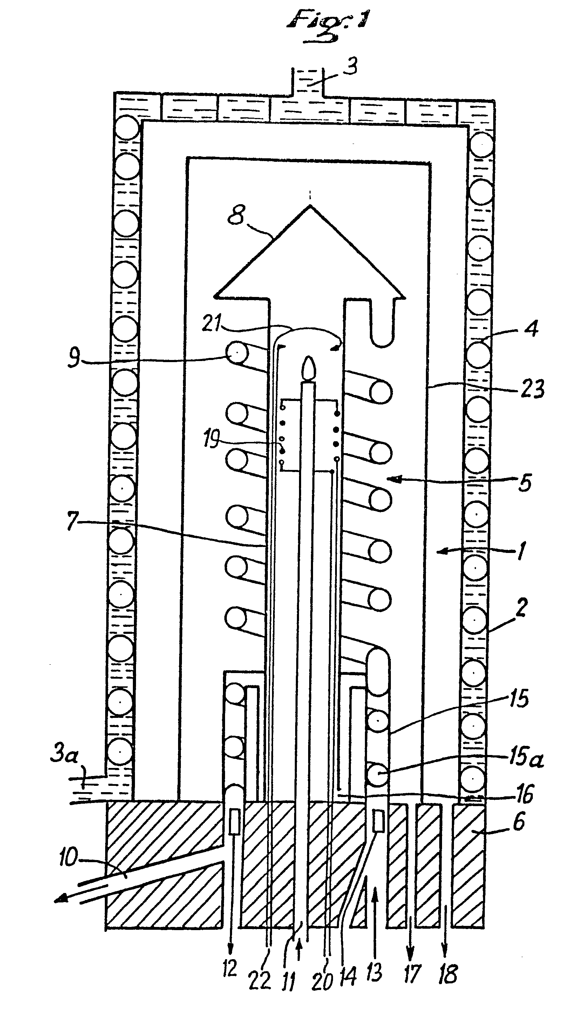

la figure 1 représente le calorimètre en coupe axiale et

la figure 2 un schéma général de l'installation.

Le calorimètre comporte deux cellules annulaires et concentriques. La cellule extérieure de référence 1 possède une double paroi extérieure 2, dans laquelle circule l'eau d'échange externe introduite par une conduite 3, guidée en spirale au moyen d'une chicane 4 logée dans la double paroi et évacuée par une conduite 3a. Cette eau sert à maintenir la température de la paroi externe de la cellule 1 à une valeur de référence constante.

La cellule intérieure de mesure 5 est séparée de la cellule de référence 1 par une paroi annulaire 23, les deux cellules étant placées sur un socle 6.

Dans l'axe longitudinal de la cellule de mesure 5 est placée une cheminée tubulaire 7 dont une extrémité repose sur le socle 6 et l'autre est fermée par un capuchon 8. L'intérieur du capuchon 8 communique avec un serpentin 9 enroulé autour de la paroi extérieure de la cheminée 7 et servant à évacuer les produits de combustion vers la sortie au moyen de la conduite 10 aménagée dans le socle 6 et logeant une sonde thermique 12 permettant de mesurer la température de sortie de gaz Ts. A l'intérieur de la cheminée, débouche à sa partie supérieure l'arrivée du gaz combustible par la conduite 11 ménagée dans le socle. L'air comburant arrive par la conduite 13 logée dans le socle et renfermant une sonde de température 14 permettant de mesurer la température de l'air d'entrée Te et passe dans un échangeur de chaleur, représenté par la gaine 15, à contre-courant de gaz brûlés circulant dans un serpentin 15a, qu'il quitte par une ouverture 16 pour pénétrer à l'intérieur de la cheminée 7.

Deux canaux 17 et 18 débouchant respectivement dans la cellule 1 et dans la cellule 5 et traversant le socle 6 sont reliés à un capteur de pression différentielle permettant la mesure de $\triangle P$.

A la partie supérieure de la cheminée 7, entourant le tube 11 d'arrivée de gaz combustible, et placée une résistance électrique 19 qui reliée à une alimentation par des fils d'amenée de courant 20 sert à maintenir le flux calorifique constant.

Un éclateur d'allumage 21 alimenté par des fils d'amenée de courant 22 est placé au dessus de l'extrémité du brûleur 11.

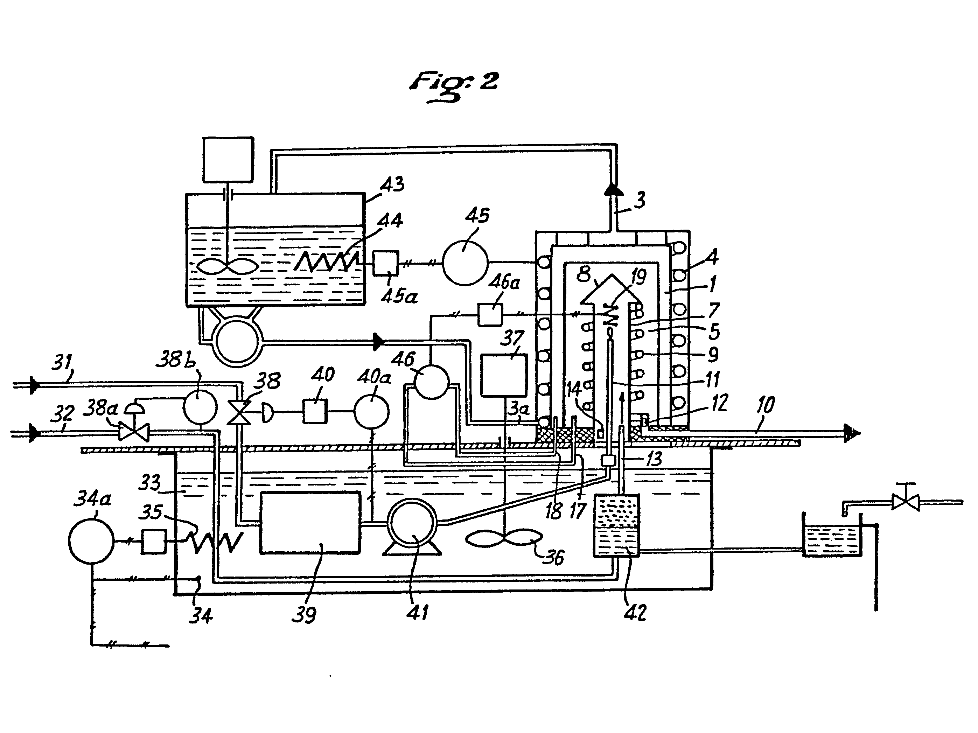

L'installation complète représentée à la figure 2 comprend le calorimètre décrit précédemment, dont la construction est simplifiée pour la clarté du dessin.

Son alimentation en gaz et en air comprend deux conduites 31 et 32 qui traversent un bain de stabilisation de température de gaz, par exemple un bain d'huile 33, dont la température est commandée au moyen d'une sonde de température 34 et d'un régulateur 34a et d'une résistance chauffante 35 et qui est brassé au moyen d'un système d'agitation 36 entraîné par un moteur 37.

Sur le circuit de gaz (conduit 31), on trouve une vanne 38 commandée par un régulateur de pression comprenant un capteur de pression 40a et un régulateur 40, un réservoir 39 et une pompe volumétrique de haute précision 41 reliée au conduit d'arrivée du gaz dans le calorimètre. La pompe 41 est une pompe rotative dont la vitesse de rotation est commandée par des moyens d'alimentation en courant stables et de précision connus, par exemple un générateur électronique.

Le circuit d'air (conduit 32) comprend une vanne 38a commandée par un régulateur de débit 38b et un humidificateur 42 assurant la saturation d'air.

Le circuit d'eau de l'échangeur externe 2 du calorimètre conduit celle-ci par la conduite 3, puis par un bac agité 43 dont la température est régulée au moyen d'une sonde de température 45 et d'un régulateur 45a mesurant la température de l'échangeur externe 2 et commandant une résistance chauffante 44 placée dans le bac 43.

La pression différentielle $\triangle P$ est mesurée au moyen d'un capteur 46 qui commande, grâce à un régulateur 46a, la résistance de chauffage 19 placée à l'intérieur de la cheminée 7.

Une unité d'électronique de conditionnement et d'acquisition, munie d'un microcalculateur, non représentée, reçoit des informations relatives à la pression de la pompe volumétrique 41, à la température du bain de stabilisation de température des gaz, à la pression différentielle $\triangle P$ des cellules 1 et 5, à la température de l'échangeur externe et aux températures d'entrée Te du brûleur et de sortie Ts des gaz brûlés et actionne les vannes de débit de gaz 38 et d'air 38a, les circuits de chauffage du bain 35, du bac d'eau 44 et de la résistance chauffante 19 placée au dessus du brûleur, en utilisant les algorithmes de régulation assurant les fonctions de régulation des organes respectifs.

Au cours de la phase d'étalonnage, on commute le gaz de référence sur l'appareil, qui en brûlant fournit une puissance calorifique de référence $q_r \cdot PCS_r$. La résistance additionnelle 19 est alimentée de telle sorte, que la puissance totale produite soit égale à une valeur prédéterminée.

Elle apporte une puissance calorifique supplémentaire $W_r$.

Lorsque l'appareil est stabilisé, on s'assure que la température Ts de gaz de sortie est sensiblement voisine ou légèrement supérieure à la température Te d'entrée de gaz selon un rapport prédéterminé voisin de 1, en modifiant la température de l'échangeur externe 2 de façon à réduire le rapport Ts/Te à la valeur voulue, compte tenu du souci d'éviter le supplément de l'eau condensée dû a la contraction du volume de gaz, dont il a été question précédemment.

Pour le gaz naturel de qualité courante et pour une température de gaz d'entrée de 40°C, on maintiendra à l'aide du circuit d'échangeur externe 2 la température de gaz de sortie aux environs de 41,5°C (rapport Ts/Te d'environ 1,027).

On observe la pression différentielle $\triangle P$, qui étant proportionelle au flux calorifique, conduit à la formule:

$$\triangle P = (q_r \cdot PCS_r + W_r) K_3.$$

Pour déterminer le pouvoir calorifique inconnu d'un gaz, on commute l'entrée des gazet pour maintenir la pression différentielle $\triangle P$ égale à la pression différentielle observée au cours de l'étalonnage, on doit agir sur la puissance $W_g$ délivrée par la résistance additionnelle 19. En d'autres mots, l'égalité des flux calorifiques émis lors de la phase d'étalonnage et de la phase de mesure est réalisée en pilotant la puissance additionnelle de la résistance 19 pour maintenir à la même valeur le signal du capteur 46 de pression différentielle.

Lorsque la stabilisation de l'appareil est atteinte, on mesure cette puissance.

La valeur du pouvoir calorifique supérieur du gaz mesuré sera calculée selon:

$$PCS_g = PCS_r + \frac{W_r - W_g}{q_g}.$$

L'invention n'est pas limitée aux modes de réalisation décrits, elle comprend également des variantes susceptibles d'être apportées par tout homme de l'art.

## Revendications

1. Procédé de mesure en continu du pouvoir calorifique supérieur d'un gaz combustible au moyen d'un calorimètre comportant autour d'une cheminée centrale logeant un brûleur et des moyens d'alimentation du brûleur à pression et débit déterminés, deux cellules annulaires et concentriques (1, 5), la paroi externe (2) de la cellule externe (1) étant pourvue en moyens d'échange de chaleur pour la régulation de sa température, lesquelles cellules concentriques séparées par une cloison annulaire (23) parallèle aux parois de cellules sont reliées à un capteur de pression différentielle (46), caractérisé en ce qu'on maintient le rapport de la température de sortie Ts de gaz brûlés à celle Te de gaz d'entrée à une valeur sensiblement voisine de 1 et le degré de saturation de l'air comburant d'alimentation du brûleur à la limite de saturation en eau, de façon à prendre en compte en totalité la chaleur latente de condensation d'eau formée par la combustion et à mesurer le flux calorifique total émis par la combustion du gaz.

2. Procédé selon la revendication 1, caractérisé en ce qu'on maintient la température de sortie Ta de gaz brûlés à une valeur légèrement supérieure à celle de gaz d'entrée de l'ordre d'un ou deux degrés, pour que, malgré la contraction du volume de gaz à la suite de la combustion, la totalité de la vapeur d'eau apporté par l'air comburant saturé soit conservée à l'état de vapeur dans les gaz de sortie sans subir de condensation et ne se trouve condensée que la quantité exacte d'eau formée par la combustion.

3. Procédé selon la revendication 1 ou 2, caractérisé en ce qu'on alterne les périodes de mesure du pouvoir calorifique du gaz échantillon $PCS_g$ avec les périodes d'étalonnage de mesure du pouvoir calorifique $PCS_r$ connu d'un gaz de référence, en effectuant les mesures des flux calorifiques selon une méthode de zéro, en ramenant pour chaque mesure la valeur du flux calorifique à une valeur constante de consigne par apport d'une énergie calorifique supplémentaire $W_r$ et $W_g$, de façon à ce que, les débits de gaz $q_g$ et $q_r$ étant constants, en égalisant les flux calorifiques émis en phase d'étalonnage et en phase de mesure selon l'équation:

$$q_r \cdot PCS_r + W_r = q_g \cdot PCS_g + W_g,$$

la valeur du pouvoir calorifique supérieur du gaz échantillon

$$PCS_g = PCS_r + \frac{W_r - W_g}{q_g}$$

s'exprime en fonction de valeurs mesurées d'énergies calorifiques supplémentaires d'apport extérieur $W_r$ et $W_g$, s'affranchissant ainsi des erreurs dues aux non linéarités du capteur de pression différentielle.

4. Procédé selon l'une des revendications 1 à 3, caractérisé en ce que dans la phase d'étalonnage on amène la valeur du flux total émis par la combustion de gaz de référence à une valeur de consigne par apport complémentaire d'énergie calorifique, en maintenant le rapport des températures de sortie de gaz brûlés et de gaz d'entrée à une valeur constante par les moyens d'échange de chaleur dont est pourvue la paroi externe (2) de la cellule externe (1) et que dans la phase de mesure, on ramène la valeur du flux total émis à la même valeur de consigne par apport complémentaire d'énergie calorifique.

5. Procédé selon la revendication 4, caractérisé en ce que l'égalité des flux calorifiques réalisée au cours de l'étalonnage et au cours de la mesure du gaz échantillon est contrôlée par les mesures de pressions différentielles $\triangle P$ au moyen d'un capteur (46) relié aux deux cellules (1, 5) du calorimètre, de façon à maintenir $\triangle P$ à une valeur constante de consigne au cours de ces deux mesures.

6. Installation de mesure en continu du pouvoir calorifique supérieur d'un gaz combustible, comprenant un calorimètre comportant autour d'une cheminée centrale (7) logeant un brûleur (11) et des moyens d'alimentation du brûleur en gaz combustible et en air (13) comburant à pressions et débits déterminés, deux cellules annulaires et concentriques (1, 5), la paroi externe (2) de la cellule externe (1) étant pourvue de moyens d'échange de chaleur, lesdites cellules concentriques séparées par une cloison annulaire (23) parallèle aux parois de cellules sont reliées à un capteur de pression différentielle (46), pour la mise en oeuvre du procédé selon l'une des revendications 1 à 5, caractérisée en ce que la cheminée centrale (7) est fermée au sommet où débouche l'entrée d'un serpentin (9) qui entoure la paroi externe de la cheminée (7) et débouche à son extrémité opposée à l'extérieur du calorimètre pour l'évacuation (10) des gaz brûlés, une sonde de température (12) étant logée au contact de gaz brûlés, une sonde de température (14) au contact des gaz d'entrée, les signaux de mesure de ces sondes étant transmis à une unité de calcul qui actionne un circuit de chauffe (44) de l'échangeur de chaleur dont est pourvue la paroi externe (2) de la cellule externe (1).

7. Installation selon la revendication 6, caractérisée en ce qu'une pompe volumétrique (41) reliée, d'une part à une conduite d'arrivée du gaz (31) de référence et du gaz échantillon, munie d'un régulateur à pression constante (40) et, d'autre part, au brûleur (11) du calorimètre et un humidificateur (42) d'air relié, d'une part, à une conduite (32) d'arrivée d'air munie d'un régulateur de débit (38b) et, d'autre part, à la cheminée centrale (7) du calorimètre, sont plongées dans un bain thermostaté (33).

8. Installation selon la revendication 6 ou 7, caractérisée en ce qu'une résistance électrique de chauffe (19) est logée dans la cheminée (7) du calorimètre et en ce que les deux cellules (1, 5) du calorimètre sont reliées par des tubulures (17, 18) à un capteur de pression différentielle (46) associé à une unité de calcul qui actionne un circuit de commande de la puissance fournie à ladite résistance électrique de chauffe.

9. Installation selon l'une des revendications 6 à 8, caractérisée en ce que le tube (11) alimentant le brûleur en gaz combustible débouche à la partie supérieure de la cheminée centrale (7).

10. Installation selon l'une des revendications 6 à 9, caractérisée en ce que la partie inférieure du serpentin (9) traverse un échangeur de chaleur (15) à contre-courant de l'air d'alimentation du brûleur.

11. Installation selon l'une des revendications 6 à 10, caractérisée en ce que la pompe volumétrique (41) est commandée par un système d'alimentation électrique stable et de précision connue.

## Patentansprüche

1. Verfahren für die kontinuierliche Messung der Heizwerte brennbarer Gase mittels eines Heizwertmessers, bei dem um einen zentralen Kamin, in dem der Brenner und die Speisungseinrichtungen des Brenners mit vorbestimmtem Druck und Durchsatz untergebracht sind, zwei ringförmige konzentrische Zellen (1, 5) angeordnet sind, wobei die Aussenwand (2) der äusseren Zelle (1) mit Wärmetauschermitteln für die Regelung ihrer Temperatur versehen ist und die durch eine parallel zu den Zellenwänden verlaufende ringförmige Wand (23) getrennten konzentrischen Zellen an einen Differentialdruckgeber angeschlossen sind, dadurch gekennzeichnet, dass das Verhältnis zwischen der Auslasstemperatur Ts der verbrannten Gase und der Einlasstemperatur Te der Gase auf einem Wert gehalten wird, der sehr nahe bei 1 liegt, während der Sättigungsgrad der Verbrennungsluft des Brenners an der Grenze der Sättigung mit Wasser gehalten wird, um die gesamte latente Wärme der Wasserkondensation zu berücksichtigen, die durch die Verbrennung verursacht wird und um die gesamte durch die Verbrennung des Gases verursachte Abwärme zu messen.

2. Verfahren nach Anspruch 1, dadurch gekennzeichnet, dass die Auslasstemperatur Ts der verbrannten Gase auf einem Wert gehalten wird, der um etwa 1 oder 2 Grad über demjenigen des Einlassgases gehalten wird, damit trotz der Komprimierung des Gasvolumens aufgrund der Verbrennung der gesamte Umfang des durch die gesättigte Verbrennungsluft eingebrachten Wasserdampfes in den Auslassgasen als Dampf erhalten bleibt, ohne kondensiert zu werden und dass nur die genaue Menge Wasser kondensiert wird, die durch die Verbrennung entsteht.

3. Verfahren nach einem der Ansprüche 1 oder 2, dadurch gekennzeichnet, dass die Messperioden des Heizwertes der Gasprobe $PCS_g$ mit den Perioden der Eichmessung des Heizwertes $PCS_r$ abgewechselt werden, die von einem Bezugsgas bekannt sind, wobei die Messung des Wärmeflusses nach einer Null-Methode durchgeführt wird, indem man bei jeder Messung den Wert des Wärmeflusses durch Zugabe einer zusätzlichen Wärmeenergie $W_r$ und $W_g$ auf einen konstanten Vorgabewert bezieht, so dass, wenn der Gasdurchsatz $q_g$ und $q_r$ konstant ist, und

wenn man den in der Eichpase und in der Messphase erhaltenen Wärmefluss nach der Gleichung

$$q_r \cdot PCS_r + W_r = q_g \cdot PCS_g + W_g,$$

ausgleicht, der Heizwert der Gasprobe

$$PCS_g = PCS_r + \frac{W_r - W_g}{q_g}$$

nach von aussen zugeführten zusätzlichen gemessenen Wärmeenergien $W_r$ und $W_g$ ausgedrückt wird, um so Fehler auszuschliessen, die auf einer mangelnden Linearität des Differentialdruckgebers beruhen.

4. Verfahren nach einem der Ansprüche 1 bis 3, dadurch gekennzeichnet, dass während der Eichpase der durch die Verbrennung des Bezugsgases abgegebene gesamte Wärmefluss durch ergänzende Zugabe von Wärmeenergie auf einen Vorgabewert gebracht wird, indem man das Verhältnis zwischen Auslasstemperatur der verbrannten Gase und Einlasstemperatur der Gase durch Wärmetauschermittel, mit denen die Aussenwand (2) der äusseren Zelle (1) ausgerüstet ist, auf einem konstanten Wert hält, und indem man in der Messphase den Wert des gesamten abgegebenen Wärmeflusses durch Zugabe zusätzlicher Wärmeenergie auf den gleichen Vorgabewert bringt.

5. Verfahren nach Anspruch 4, dadurch gekennzeichnet, dass die Übereinstimmung der in der Eichphase und in der Messphase erhaltenen Wärmeflüsse mittels eines an die zwei Zellen (1, 5) des Heizwertmessers durch differentielle Druckmessungen $\triangle P$ kontrolliert werden, damit der Wert $\triangle P$ im Laufe der beiden Messungen auf einem konstanten Vorgabewert gehalten wird.

6. Einrichtung für die kontinuierliche Messung des Heizwertes eines brennbaren Gases mit einem Heizwertmesser, der um einen zentralen Kamin (7), in dem ein Brenner (11) und die Einspeisungsmittel für brennbares Gas und Luft (13) mit vorbestimmtem Druck und Durchsatz angeordnet sind, zwei ringförmige konzentrische Zellen (1, 5) aufweist, wobei die Aussenwand (2) der äusseren Zelle (1) mit Wärmetauschermitteln ausgerüstet ist, wobei die durch eine parallel zu den Wänden der Zellen verlaufende ringförmige Wand (23) getrennten konzentrischen Zellen an einen Differentialdruckmesser (46) angeschlossen sind, um das Verfahren nach einem der Ansprüche 1 bis 5 durchzuführen, dadurch gekennzeichnet, dass der zentrale Kamin (7) an seiner Oberseite, an der eine die Aussenwand des Kamins (7) umhüllende Rohrschlange (9) austritt, geschlossen ist, wobei diese Rohrschlange mit ihrem anderen Ende an der Aussenseite des Heizwertmessers für die Abführung (10) der verbrannten Gase austritt, und ein Temperaturfühler (12) in Berührung mit den verbrannten Gasen angeordnet ist und ein weiterer Temperaturfühler mit den Einlassgasen in Berührung steht, deren Messsignale an eine Rechnereinheit geliefert werden, die den Heizkreis (44) des Wärmetauschers einschaltet, der an der Aussenwand (2) der äusseren Zelle (1) angeordnet ist.

7. Einrichtung nach Anspruch 6, dadurch gekennzeichnet, dass eine Verdrängungspumpe (41), die einerseits an eine Zuleitung (31) für das Bezugsgas oder die Gasprobe angeschlossen ist und einen konstanten Druckregler (40) aufweist und, andererseits, an den Brenner (11) des Heizwertmessers, und ein Luftbefeuchter (42), der einerseits an eine mit einem Durchsatzregler (38b) ausgerüstete Luftzuleitung (32) und, andererseits, an den zentralen Kamin (7) des Heizwertmessers angeschlossen ist, in ein Temperaturbad (33) eintauchen.

8. Einrichtung nach Anspruch 6 oder 7, dadurch gekennzeichnet, dass ein elektrischer Heizwiderstand (19) in dem Kamin (7) des Heizwertmessers angeordnet ist, und dass die beiden Zellen (1, 5) des Heizwertmessers durch Rohrleitungen (17, 18) an einen Differentialdruckgeber (46) angeschlossen sind, der mit einer Rechnereinheit in Verbindung steht, welcher den Steuerkreis für die an den elektrischen Heizwiderstand gelieferte Leistung betätigt.

9. Einrichtung nach einem der Ansprüche 6 bis 8, dadurch gekennzeichnet, dass das den Brenner mit brennbarem Gas versorgende Rohr (11) am Oberteil des zentralen Kamins (7) austritt.

10. Einrichtung nach einem der Ansprüche 6 bis 9, dadurch gekennzeichnet, dass der untere Teil der Rohrschlange (9) einen Wärmetauscher (15) gegenläufig zur Speiseluft des Brenners durchquert.

11. Einrichtung nach einem der Ansprüche 6 bis 10, dadurch gekennzeichnet, dass die Verdrängungspumpe (41) durch ein stabiles Stromversorgungssystem bekannter Genauigkeit gesteuert wird.

**Claims**

1. A method for the continuous measurement of the higher calorific value of a combustible gas by means of a calorimeter comprising, about a central chimney housing a burner and means for supplying the burner at given pressure and flow rate, two annular concentric cells (1, 5), the external wall (2) of the external cell (1) being provided with heat exchange means for regulating its temperature, which concentric cells separated by annular dividing wall (23) parallel to the cell walls are connected to a differential pressure sensor (46), characterized in that the ratio of the outgoing temperature Ts of the burnt gases to that Te of the incoming gases is kept at a value substantially close to 1 and the degree of saturation of the combustive air feeding the burner is kept at the water saturation limit, so as to take into account the whole of the latent heat of condensation of water formed by the combustion and to measure the total heat flow emitted by combustion of the gas.

2. The method as claimed in claim 1, characterized in that the outgoing temperature Ts of the burnt gases is kept at a value slightly greater than that of the incoming gases within one or two degrees, so that, despite the contraction of the gas volume following combustion, the whole of the water vapour brought by the saturated combustive air is kept in the vapor state in the outgoing gases without undergoing condensation and only the exact amount of water formed by the combustion is condensed.

3. The method as claimed in claim 1 or 2, characterized in that the periods of measuring the calorific value of the sample gas $PCS_g$ are alternated with the periods of calibration for measuring the known calorific value $PCS_r$ of a reference gas, by carrying out the measurements of the heat flows using a zero method, by bringing the value of the heat flow or each measurement to a constant reference value with addition of heat energy $W_r$ and $W_g$, so that, with the gas flow rates $q_g$ and $q_r$ constant, by equilizing the heat flows emitted during the calibration phase and during the measurement phase in accordance with the equation:

$$q_r \cdot PCS_r + W_r = q_g \cdot PCS_g + W_g,$$

the value of the higher calorific value of the sample gas

$$PCS_g = PCS_r + \frac{W_r - W_g}{q_g}$$

is expressed as a function of measured values of additional external heat energy supplies $W_r$ and $W_g$, thus eliminating errors due to the non linearities of the differential pressure sensor.

4. The method as claimed in one of claims 1 to 3, characterized in that, in the calibration phase, the value of the total flow emitted by combustion of the reference gas is brought to a reference value by addition of complementary heat energy, while maintaining the ratio of the temperatures of the outgoing burnt gases and of the incoming gas at a constant value via the heat exchange means with which the external wall of the external cell is provided and, in the measurement phase, the value of the total flow emitted is brought to the same reference value by supplying complementary heat energy.

5. The method as claimed in claim 4, characterized in that the equality of the heat flows provided during calibration and during measurement of the sample gas is controlled by the differential pressure measurements $\triangle P$ by means of a sensor connected to two cells of the calorimeter so as to maintain $\triangle P$ at constant reference value during these two measurements.

6. An installation for the continuous measurement of the higher calorific value of a combustible gas, comprising a calorimeter with, about a central chimney (7) housing a burner (11) and means for supplying the burner with combustible gas and combustive air (13) at given pressures and flow rates, two annular concentric cells (1, 5), the external wall (2) of the external cell (1) being provided with heat exchange means, said concentric cells separated by an annular dividing wall (23) parallel to the cell walls are connected to a differential pressure sensor (46), for implementing the method as claimed in one of claims 1 to 5, characterized in that the central chimney (7) is closed at the top where the inlet of a coiled tube (9) emerges which surrounds the external wall of the chimney (7) and whose other end emerges outside the calorimeter for discharging (10) the burnt gases, a temperature probe (12) being disposed in contact with the burnt gases, a temperature probe (14) in contact with the input gases, the measurement signals from these probes being transmitted to a computing unit which actuates a circuit (44) for heating the heat exchanger with which the external wall (2) of the external cell (1) is provided.

7. The installation as claimed in claim 6, characterized in that a displacement pump (41) connected, on the one hand to an intake duct (31) for the reference gas and for the sample gas, having a constant pressure regulator (40), and on the other hand, to the burner (11) of the calorimeter and an air humidifier (42) connected, on the one hand, to an air intake duct (32) having a flow rate regulator (38b) and, on the other hand, to the central chimney (7) of the calorimeter are plunged in a thermostat controlled bath.

8. The installation as claimed in claim 6 or 7, characterized in that a heating electric resistance (19) is housed in the chimney (7) of the calorimeter and the two cells (1, 5) of the calorimeter are connected by pipes (17, 18) to a differential pressure sensor (46) associated with a computing unit which actuates a circuit controlling the power supplied to said electric heating resistance.

9. The installation as claimed in one of claims 6 to 8, characterized in that the tube (11) feeding the burner with the combustible gas opens into the upper part of the central chimney (7).

10. The installation as claimed in one of claims 6 to 9, characterised in that the lower part of the coiled tube (9) passes through a heat exchanger (15) countercurrent wise with respect to the air feeding the burner.

11. The installation as claimed in one of claims 6 to 10, characterized in that said displacement pump (41) is controlled by a stable electric supply system of known precision.

## Fig. 1

Fig. 2

EP 0 210 081 B1